# EUROPEAN PATENT SPECIFICATION

(11) **EP 1 819 368 B1**
(45) Date of publication and mention of the grant of the patent: **21.03.2012**
(21) Application number: 05818891.3
(22) Date of filing: 25.11.2005
(51) Int. Cl.: A61L 2/20, A61L 2/07, A61L 2/16

(54) **HYDROGEN PEROXIDE VAPOR STERILIZER AND STERILIZING METHODS USING THE SAME**
WASSERSTOFFPEROXID-DAMPFSTERILISATOR UND STERILISIERVERFAHREN DAMIT
STERILISATEUR A VAPEUR DE PEROXYDE D'HYDROGENE ET PROCEDES DE STERILISATION METTANT EN OEUVRE UN TEL STERILISATEUR

(30) Priority: 26.11.2004 KR 20040098050; 14.12.2004 KR 20040105319
(43) Date of publication of application: 22.08.2007
(73) Proprietor: Human Meditek Co., Ltd, Seoul 150-023 (KR)
(72) Inventor: KO, Jung Suek, Jongno-gu, Seoul 110-524 (KR)
(74) Representative: Maser, Jochen
(86) International application number: PCT/KR2005/003989
(87) International publication number: WO 2006/057523

(56) References cited:
- JP-A- 2002 022 101
- KR-A- 2003 084 585
- US-A- 5 482 684
- US-A- 5 667 753
- US-B1- 6 495 100
- US-B1- 6 656 426
- US-B2- 6 734 405

## Description

### Technical Field

The present invention relates to a method for sterilizing articles by killing microbes with hydrogen peroxide. More particularly, the present invention relates to a method in which the vapor formed by heating a hydrogen peroxide solution in a vacuum condition is brought into contact with the articles.

### Background Art

To sterilize articles which must be sterilized to be used in particular purposes (hereinafter referred to just as "articles"), such as various medical tools or instruments (disposables inclusive), a hydrogen peroxide solution is widely used to kill microbes living thereupon. Many techniques in regard to this have been suggested, and a few representatives of them will be described bellow.

The US patent US 6,734,405 B2 discloses a method for sterilizing an article with hydrogen peroxide in a reaction vessel, whereby the hydrogen peroxide solution vaporizes as it contacts the wall surface of a bore in an electrically insulative housing outside of the reaction vessel and is then injected as hydrogen peroxide vapor in the reaction vessel, in which the article to be sterilized is placed.

Korean Pat. No. 0132233, titled "Hydrogen Peroxide Plasma Sterilization System", discloses that after articles are pretreated by coming into contact with a hydrogen peroxide solution, microbes are killed with the active species generated from hydrogen peroxide plasma, and the hydrogen peroxide remaining in the articles is dissociated into non-toxic chemicals.

Korean Pat. No. 10-0351014, entitled "Vapor Sterilization Method Using Non-Aqueous Hydrogen Peroxide Source, Apparatus therefor and Non-Aqueous Hydrogen Peroxide Complex," discloses a container in which an article is contacted with hydrogen peroxide vapor supplied from a source comprising a substantially non-aqueous hydrogen peroxide complex. Also, the container is equipped with a heater for heating the complex to produce vapor from the complex.

Because a concentration of hydrogen peroxide higher than 60% is prohibited by government regulations from being applied to articles, such a sterilization system as is described above generally employs a concentration of hydrogen peroxide from 50 to 58%. However, the sterilizing power obtained using such a low concentration of hydrogen peroxide is unsatisfactory. As an alternative to avoid this problem, a vapor sterilization method has been developed featuring the use of a complex obtained by concentrating an aqueous hydrogen peroxide solution (e.g., removing water from an aqueous hydrogen peroxide solution).

Typically, a conventional vapor sterilization system is structured to produce and diffuse hydrogen peroxide vapor inside or outside a reaction vessel and to generate plasma, which is then brought into contact with an article. In such a system, the sterilization is conducted under low pressure conditions in the reaction vessel. Under these conditions, thus, hydrogen peroxide vapor plasma, which plays a pivotal role in sterilization, has difficulty in penetrating into tools having narrow and long lumens, for example, endoscopes having a tube 50 cm long with a diameter of 1 mm or less. That is, tools having narrow, long cavities are not completely sterilized using conventional hydrogen peroxide vapor sterilizers.

The reason for the imperfect sterilization is that, since water is usually lighter in molecular weight and thus higher in vapor pressure than hydrogen peroxide, water vapor occupies narrow, long cavities, such as lumens, prior to hydrogen peroxide vapor, upon sterilization.

In addition, direct contact with hydrogen peroxide vapor plasma according to conventional plasma sterilization methods causes polymeric medical tools to undergo a color change or a property change such as setting. When articles occupy 70% or more of the volume of a reaction vessel, some of them are highly apt to remain non-sterilized after sterilization in the conventional sterilization systems.

Further, the reaction vessel of conventional systems is significantly limited in regard to size because plasma must be generated uniformly over the internal space of the reaction vessel. Also, a cathodic self-bias, potentially occurring upon the generation of plasma, is likely to cause nearby articles to remain non-sterilized.

Fundamentally structured to sterilize articles within a reaction vessel by supplying hydrogen peroxide vapor thereto or by generating hydrogen peroxide plasma for the production of reactive species responsible for killing microbes, conventional systems cannot perform sterilization to perfection when articles are highly bulky in volume. Additionally, hydrogen peroxide may be discharged without being completely decomposed into oxygen, hydrogen and water, producing air pollution, which can cause respiratory ailments in the user or patients.

Most conventional sterilization systems are equipped with cathode and anode for generating plasma in reaction vessels. Before articles are put into the reaction vessels, the systems are required to maintain the temperature of the reaction vessels at 30°C or higher to obtain a maximum sterilization effect, as disclosed in U.S. Pat. No. 6,365,102. This patent suggests a method comprising evacuating a chamber, generating plasma in the chamber, venting the chamber to approximately atmospheric or subatmospheric pressure, and repeating the evacuating, generating plasma, and venting at least two times.

In addition, the aforementioned patent states since the energy of plasma is radiated too insufficiently in a vacuum condition to increase the temperature of the reaction vessel within a short time period, the air introduced into the reaction vessel serves as an effective medium for transferring the energy of plasma, thereby increasing the temperature of the reaction vessel.

Although characterized in that the temperature of the reaction vessel is maintained at 30°C in an early stage to readily evaporate an aqueous hydrogen peroxide solution and not to condense the hydrogen peroxide vapor, the sterilization of the above-mentioned U.S. patent performs sterilization in the same manner as in conventional techniques.

The low-pressure condition required to the sterilization method of the U.S. patent does not guarantee the sterilization of an article having a narrow, long lumen, e.g., a flexible endoscope which is 50 cm long with Φ1mm or less, because hydrogen peroxide vapor plasma, essentially responsible for killing microbes, does not penetrate well into the lumen.

### Disclosure of the Invention

Accordingly, the present invention has been made keeping in mind the above problems occurring in the prior art, and an object of the present invention is to provide a method for effectively sterilizing an article using hydrogen peroxide vapor.

In order to accomplish the above object, the present invention provides a method for sterilizing an article in a reaction vessel using hydrogen peroxide vapor as a sterilant, comprising: maintaining an inner space of the reaction vessel at a vacuum pressure lower than an equilibrium vapor pressure of hydrogen peroxide; heating the inner space of the reaction vessel to a temperature between the range from 30 to 60 °C; injecting a hydrogen peroxide solution into the inner space of the reaction vessel; thermally vaporizing the hydrogen peroxide solution within the reaction vessel at the same time of the injection step; sealing the inner space of the reaction vessel and maintaining the article in a hydrogen peroxide vapor atmosphere; and introducing external gas into the inner space of the reaction vessel to increase the pressure of the inner space to a predetermined value not more than the atmospheric pressure.

In the method, the external gas is further introduced to a pressure from 13.33 kPa to 79.99 kPa (100 to 600 torr). Preferably, the external gas is heated before the introducing step.

In the method of the present invention, the heating step preferably comprises the use of a radiating heating means 50 for heating the inner space of the reaction vessel. Preferably, the radiating heating means is an infrared lamp or a halogen lamp. Also, the radiating heating means may radiate heat energy in an intermittent manner.

In the method of the present invention, the introducing step is conducted multiple times to increase the pressure of the inner space in a stepwise manner.

In accordance with a modification of the present invention, the method may further comprise: decreasing the pressure of the inner space to the predetermined vacuum pressure of the maintaining step or less; and re-increasing the pressure of the inner space to the atmospheric pressure or less, after the introducing step. In this modification, the method may further comprises repeating pressure decrease and pressure re-increase at least once more, after the re-increasing step.

In the method of the present invention, the external gas may be air.

### Brief Description of the Drawings

The above and other objects, features and other advantages of the present invention will be more clearly understood from the following detailed description taken in conjunction with the accompanying drawings, in which:
FIG. 1 is a schematic view showing the structure of a hydrogen peroxide vapor sterilizer ;
FIG. 2 is a pressure diagram of a reaction vessel illustrating the hydrogen peroxide vapor sterilization process of the present invention;
FIGS. 3 to 6 are pressure diagrams showing the hydrogen peroxide vapor sterilization processes in accordance with embodiments of the present invention; and
FIG. 7 is a schematic view showing the structure of hydrogen peroxide vapor sterilizer according to another embodiment .

### Best Mode for Carrying Out the Invention

Reference should now be made to the drawings, in which the same reference numerals are used throughout the different drawings to designate the same or similar components.

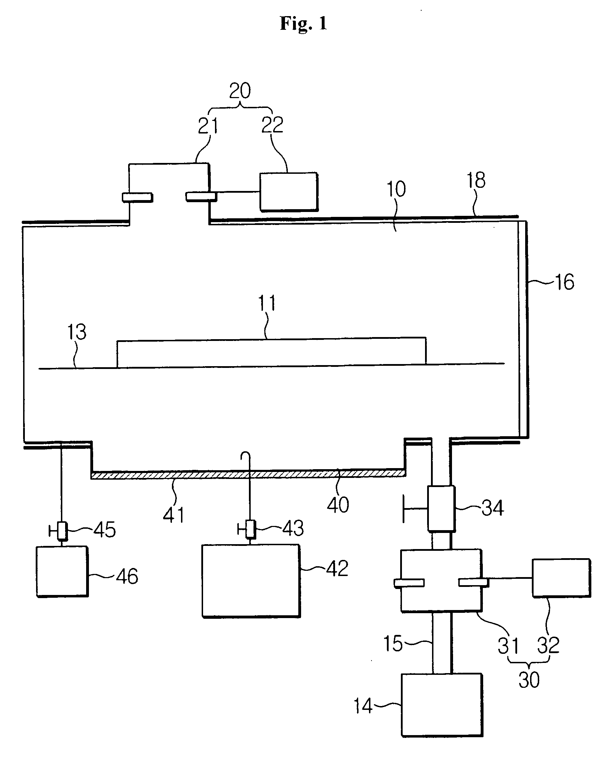

FIG. 1 is a schematic structural view showing a hydrogen peroxide vapor sterilizer applicable for sterilization using hydrogen peroxide plasma, in accordance with the present invention.

The hydrogen peroxide vapor sterilizer , as shown in this figure, comprises a reaction vessel 10 consisting of a chamber, having therein a tray 13 for supporting an article 11 such as a medical utensil or surgical tool. Usually, the article 11 is put on the tray 13 while being wrapped with a packing material. The reaction vessel 10 is vacuumed by a vacuum pump 14, connected via a discharge line 15 to a lower position of the reaction vessel 10. A door 16 is provided on one side of the reaction vessel 10.

A plasma generator 20, provided outside the reaction vessel 10, consists of a plasma chamber 21 in which two electrodes are installed facing each other, with optimal plasma generated therebetween, and a power supply 22 electrically connected to the plasma chamber, for supplying a high voltage to the electrodes.

At the discharge line 15, a plasma treatment unit 30 is provided for allowing the hydrogen peroxide vapor inside the reaction vessel 10 to pass plasma. This plasma treatment unit 10 has almost the same structure as in the plasma generator 20, with the exception that a plasma chamber 31 is installed on the discharge line 15 while a power supply 32 is electrically connected to the plasma chamber 31 so as to allow the chamber 31 to generate optimal plasma. Thus, a detailed description therefor is omitted. An automatic pressure-control valve 34 is provided on the discharge line 15 between the reaction vessel 10 and the plasma chamber 31.

For the plasma generation of the plasma generator 20 and the plasma treatment unit 30, various techniques, such as arc discharge, RF discharge, etc., may be applied, in the presence of an electric field using high-voltage DC or AC current.

Also, the reaction vessel 10 has an evaporator 40 provided on an indented internal bottom thereof and a heater 41 beneath the bottom thereof. An external hydrogen peroxide reservoir 42 is provided for supplying an aqueous hydrogen peroxide solution to the evaporator 40, with a connection therebetween. Because hydrogen peroxide is supplied from the hydrogen peroxide reservoir 42 to the evaporator 40 after the reaction vessel 10 is vacuumed, any may be used as the hydrogen peroxide reservoir 42 if it is structured to take advantage of the pressure difference in the supply of hydrogen peroxide. A flow-control valve 43, with which the flow rate of hydrogen peroxide from the reservoir to the evaporator can be controlled, is provided between the evaporator 40 and the hydrogen peroxide reservoir 42.

Provided, at the chamber for releasing the vacuum condition of the chamber, a pressure-release valve 45 is equipped with a filter through which air is filtered before entering the chamber.

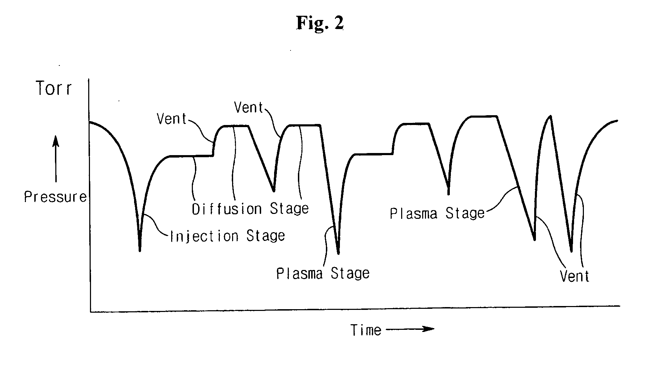

In FIG. 2, a pressure diagram of the reaction vessel according to the present invention is shown to illustrate the sterilization process using hydrogen peroxide. With reference to this pressure diagram, the sterilization process of the present invention will be described in detail.

First, electric power is applied to the heaters 41 and 18 installed respectively at the evaporator 40 and outside the reaction vessel 10, to increase the temperature of the evaporator 40, as well as that of the reaction vessel, to 10 to 30°C or higher. In the present invention, heating the reaction vessel and the evaporator may not precede other process steps, but may be conducted at a proper point of time as will be described later. For example, the evaporator is heated after or before supply of the hydrogen peroxide.

Next, an article, such as a medical utensil or a surgical tool, wrapped with a packing sheet is placed on the tray, followed by closing the door 16. At this time, the control valves 43, 45 respectively provided between the reaction vessel 10 and the hydrogen peroxide reservoir 42 and between the reaction vessel 10 and the filter 46 as well as the automatic pressure-control valve 34 installed on the discharge line 15 between the reaction vessel 10 and the plasma treatment unit 30 are in a close state. Any packing sheet can be used if it allows air penetration therethrough like cloth.

After the automatic pressure-control valve 34 installed on the discharge line 15 is opened, the operation of the vacuum pump 14 allows air to be drawn out of the reaction vessel 10 and the plasma chamber 21 to a predetermined pressure (39.99 Pa 800x10⁻³ Torr) or less). At this time, no electric field is applied from power supply 22 to the electrodes of the plasma generator 20 nor to the plasma treatment unit 30 installed on the discharge line 15. When a predetermined pressure is formed inside the reaction vessel 10 by the vacuum pump 14, the automatic pressure-control valve 34 on the discharge line 15 is closed.

Subsequently, the flow-control valve 43 is opened to supply an aqueous hydrogen peroxide solution from the hydrogen peroxide reservoir 42 to the evaporator 40 within the reaction vessel 10. A pressure difference attributed to the vacuumed reaction vessel may cause hydrogen peroxide to be spontaneously introduced into the reaction vessel. As soon as the aqueous hydrogen peroxide solution is injected into the reaction vessel, it is vaporized because the temperature of the evaporator 40, heated by the heater 41, is high enough to vaporize the solution. As the hydrogen peroxide vapor is diffused, it starts to contact and sterilize the article 11. At this time, the reaction vessel 10 is in a vacuum state, while the evaporator 40 has already been heated. As the aqueous hydrogen peroxide solution is vaporized, the hydrogen peroxide vapor pressure of the reaction vessel 10 gradually increases to an equilibrium vapor pressure.

After the aqueous hydrogen peroxide solution is vaporized, the total pressure of the reaction vessel 10 is maintained at 0.13 to 13.33 kPa (1 to 100 torr) according to the temperature inside the chamber. Because the temperature of the reaction vessel 10 is maintained at 30∼60°C with the aid of the external heater 18, the hydrogen peroxide vapor is not condensed, but remains as it is, continuously functioning as a sterilant.

In accordance with the present invention, the evaporation of the sterilant within the chamber shortens the penetration distance of the hydrogen peroxide vapor to the article 11. In addition, thanks to the high temperature maintained within the chamber, high hydrogen peroxide vapor pressure in the chamber is maintained and prevented from being condensed. Therefore, the present invention can exert potential sterilizing power to the article 11 in which the diffusion of hydrogen peroxide vapor is limited. Depending on the concentration of hydrogen peroxide, it usually takes about 5 min or less to complete the sterilization. However, although articles can be sterilized within 5 min, it is recommended that the sterilization operation be maintained for a predetermined period of time (about 30 min).

A condition of a controlled hydrogen peroxide vapor pressure, that is, a reaction pressure, within the reaction vessel 10 is maintained for a predetermined period of time sufficient to sterilize the article, followed by opening the pressure-release valve 45 to introduce external air purified through the filter 46 into the chamber. As soon as the internal pressure of the reaction vessel 10 reaches 100 to 600 torr during the ventilation, the pressure-release valve 45 is closed. If external air is excessively introduced to exceed the upper limit of the pressure range, the temperature of the chamber is decreased so as to cause the condensation of hydrogen peroxide vapor and water vapor. Therefore, it is preferable to control the volume of the introduced external air within the pressure range set above.

A drastic increase in the internal pressure of the reaction vessel 10 increases the pressure difference between the outside and the inside of an article 11 which has a diffusion-limited region, such as a lumen, thereby allowing the hydrogen peroxide vapor to readily diffuse into the diffusion-limited region of the article 11.

After maintaining the internal pressure of the chamber for a predetermined time period, the vacuum pump 14 is operated, with the automatic pressure controlling valve 34 on the discharge line 15 being open, so as to draw the gas (hydrogen peroxide vapor) out of the reaction vessel 10 and the plasma chamber 21 to a desired pressure 0.13 kPa (1 Torr) or less). Then, the pressure of the reaction vessel 10 is increased again to 100 Torr or higher and maintained thereat for a predetermined time period in the same manner as is described above.

When the vacuum pump 14 is operated, with the automatic pressure-control valve 34 on the discharge line 15 being open after the primary sterilization is conducted, the residual hydrogen peroxide vapor within the reaction vessel 10 is discharged through the discharge line 15 into the plasma chamber 31 of the plasma treatment unit 30 and then exhausted to the air.

Simultaneously with a decrease in the pressure of the plasma chambers 21 and 31 by the evacuation of the reaction vessel 10, the respective application of high voltage from the power supplies 22 and 32 to the electrodes of the plasma chambers 21 and 31 allows the generation of plasma between the electrodes.

When plasma is generated in the plasma chamber 21 of the plasma generator 20, the reactive species thus formed is diffused over the reaction vessel 10 to maintain a plasma atmosphere. Before being discharged from the reaction vessel to the plasma treatment unit 30, the hydrogen peroxide vapor is primarily decomposed by the reaction species filled in the reaction vessel 10.

Meanwhile, plasma is generated to form a plasma atmosphere in the plasma chamber 31 on the discharge line 15, as well. Thus, while passing through the discharge line 15 and then through the plasma chamber 31 of the plasma treatment unit 30, the residual hydrogen peroxide vapor inside the reaction vessel 10 is decomposed into non-toxic components, that is, water, oxygen molecules and hydrogen, by plasma energy. The exhaust gas consisting of such non-toxic molecules neither produces pollution of the environment nor damages the body.

The decomposition of hydrogen peroxide, although described as being performed after the completion of the primary sterilization, may be conducted in advance, that is, at the time when the gas is drawn out after an increase in the pressure of the reaction vessel.

As the residual hydrogen peroxide vapor within the reaction vessel 10 is decomposed and exhausted, the pressure of the reaction vessel 10 decreases to 13.33 Pa (100x10⁻³ Torr) or less. When a predetermined pressure is set within the reaction vessel 10 by the vacuum pump 14, the automatic pressure-control valve 34 on the discharge line 15 is closed, and the application of high-voltage power from the power supply 22, 32 to the plasma generator 20 and the plasma treatment unit 30 is blocked.

Thereafter, as described above, processes of injecting an aqueous hydrogen peroxide solution to the evaporator 40 within the reaction vessel 10, sterilizing the article, increasing the pressure of the reaction vessel 10 and maintaining a low pressure in the reaction vessel 10 may be repeated at least once more. Maintaining high and low pressure within the chamber in an alternating manner can bring about a great improvement in sterilizing diffusion-limited regions of articles.

Because low pressure is formed within the reaction vessel 10 upon the completion of sterilization, the air purified through the filter 10 is introduced into the reaction vessel 10 to the atmospheric pressure as the pressure-release valve 45 is gradually opened. Afterwards, the door 16 is opened to draw out the wrapped article 11 thus sterilized.

Contrary to the description for the concomitant operation of the plasma generator 20 and the plasma treatment unit 30 upon the discharge of residual hydrogen peroxide vapor from the reaction vessel 10, only the plasma treatment unit 30, if necessary, may be operated during the passage of the residual hydrogen peroxide vapor along the discharge line 15 through the plasma chamber 31, so as to decompose the hydrogen peroxide vapor into non-toxic components, which is legally permitted to be exhausted.

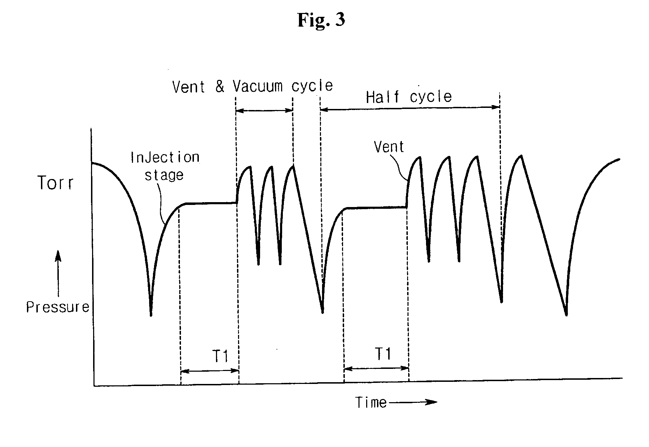

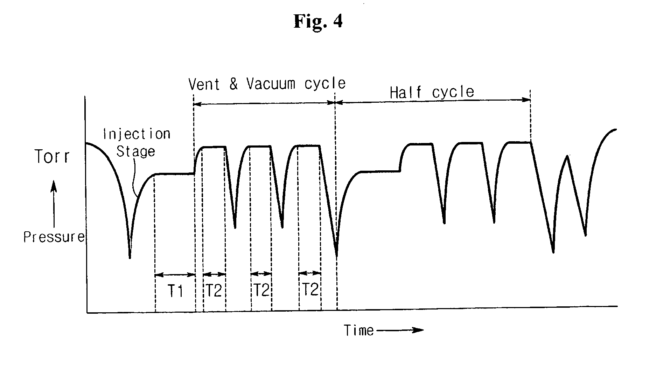

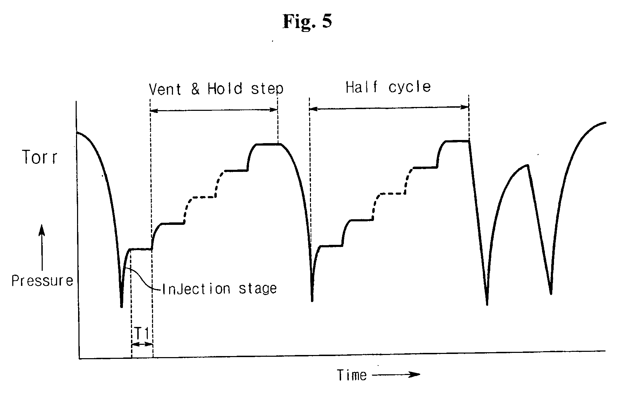

With reference to FIGS. 3 to 6, various embodiments of the sterilization method using hydrogen peroxide are illustrated. FIG. 3 is a pressure diagram of the sterilizer, obtained by injecting and vaporizing an aqueous hydrogen peroxide solution, diffusing the hydrogen peroxide vapor, maintaining a predetermined hydrogen peroxide vapor pressure for a predetermined time period and, thereafter, repeating at least once more the processes of introducing air into the chamber to a predetermined pressure, and immediately discharging the gas (hydrogen peroxide vapor) of the reaction vessel 10 to a predetermined vacuum pressure. FIG. 4 is similar to FIG. 3, with the exception of repeating once more the processes of introducing air to a predetermined pressure, maintaining this pressure for a predetermined time period, and then discharging the gas (hydrogen peroxide vapor) of the reaction vessel 10 to a predetermined vacuum pressure. FIG. 5 is a pressure diagram of the sterilizer, obtained by injecting and vaporizing an aqueous hydrogen peroxide solution, diffusing the hydrogen peroxide vapor, maintaining a predetermined hydrogen peroxide vapor pressure for a predetermined time period and, thereafter, stepwise conducting the process of introducing air to a predetermined pressure and maintaining the pressure for a predetermined time period.

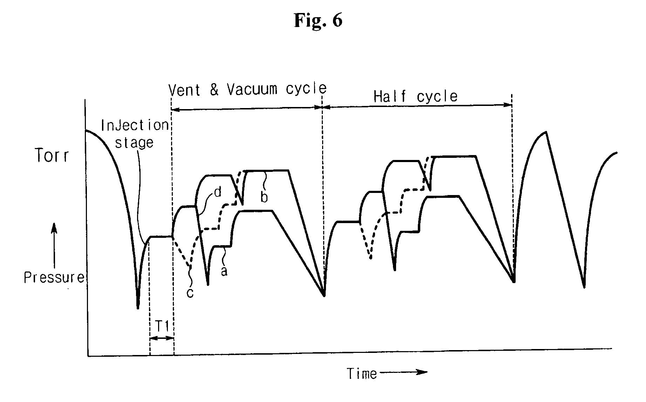

Referring to FIG. 6, the processes of introducing air into the reaction vessel 10 to a predetermined pressure and then discharging the gas (hydrogen peroxide vapor) of the reaction vessel 10 to a predetermined vacuum pressure are repeated at least once (lines a and b). The dotted line of FIG. 6 depicts the injection and vaporization of an aqueous hydrogen peroxide solution, diffusion of the hydrogen peroxide and discharge of the gas (hydrogen peroxide vapor) of the reaction vessel 10 and, thereafter, repeating the processes of air injection into the reaction vessel 10 and gas discharge from the reaction vessel 10 (line c).

In FIGS. 3 to 6, T1 and T2 independently range from 0 to 30 min and can be controlled depending on properties of the article 11.

Featuring the vaporization and diffusion of hydrogen peroxide and the injection and discharge of air, the sterilizing methods according to the embodiments described above can enhance the penetration of a sterilant into diffusion-limited regions of articles, thereby producing greatly improved sterilization effects.

### EXAMPLE 1

Sterilization effects were compared between a conventional method in which the diffusion of hydrogen peroxide is conducted alone, and the method of the present invention wherein the diffusion of hydrogen peroxide is conducted in combination with an increase in pressure (air ventilation), and the results are given in Table 1, below.

For a sterilization test, a BI (Biological Indicator), manufactured by A Company of the U.S.A., was used. "Bacillus Stearothermophilus" [Spore NO. 2.04 x 10n, n=6] served as a test microbe. The BI was applied to lumens fabricated in various dimensions, after which they were subjected to sterilization according to the methods. BI samples taken from the lumens were incubated for 48-72 hours in the same incubator and were measured for their color changes. For each case, the test was repeated 10 times and the number of satisfactory sterilizations (no growth of the microbe) was counted.

**TABLE 1**

| (unit: mm) | | | | |
|---|---|---|---|---|
| Sterilizing Process (H₂O₂ Injection) | Φ2x1000 | Φ2x2000 | Φ1x1000 | Φ1x2000 |
| Conventional-Diffusion | 10/10 | 6/10 | 0/10 | 0/10 |
| Inventive-Air Vent after Diffusion | 10/10 | 10/10 | 10/10 | 10/10 |

| | | | | |
|---|---|---|---|---|
| *No. of successful penetration/total no. of test rounds *Success in penetration: Negative response of BI sample *Fail to penetrate: Positive response of BI sample | | | | |

As seen in Table 1, good sterilization results were obtained in articles in size of Φ2x1000 by both methods, but an extreme difference in sterilization effect is found between the conventional method and the present invention as the size becomes narrower (Φ1). The difference is believed to be attributable to the fact that the pressure difference, a factor essential to the penetration of hydrogen peroxide vapor, is increased by the air introduction, thereby allowing the hydrogen peroxide vapor to readily penetrate into the lumens the size of Φ1.

After the diffusion of hydrogen peroxide vapor, the introduction of air and the evacuation of the chamber, a pressure increase through air introduction was found to further enhance the sterilization effect. It is also believed that many pressure differences can provide a more potent driving force facilitating the penetration of hydrogen peroxide vapor into diffusion-limited regions such as lumens.

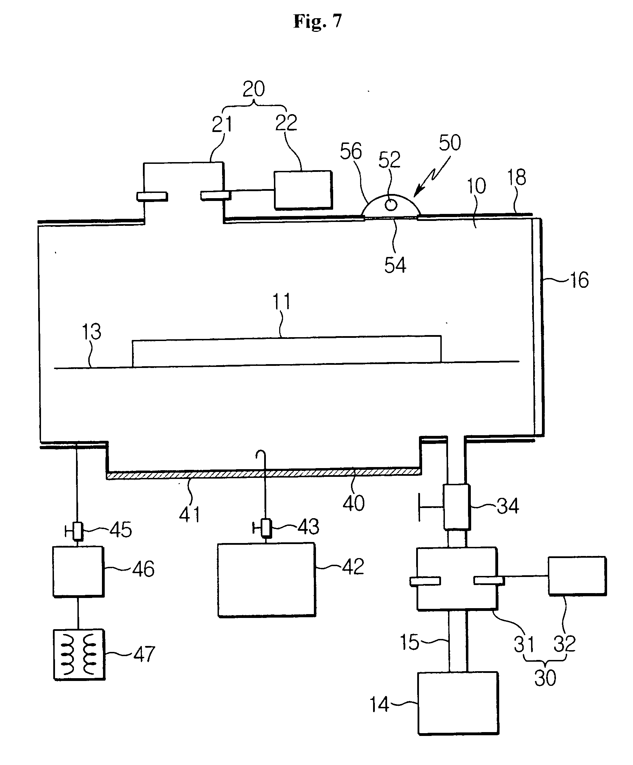

FIG. 7 is a schematic view showing the structure of a hydrogen peroxide vapor sterilizer in accordance with another embodiment of the present invention.

As seen in FIG. 7, the structure of this sterilizer is similar to that of the sterilizer of FIG. 1. The same components designated by the same reference numerals as in FIG. 1 are not further described.

Unlike the sterilizer of FIG. 3, the sterilizer according to this embodiment is equipped with a hot-air blower 47 and a radiating heater 50.

Positioned at a front end of the filter 46, the hot-air blower 47 functions to heat external air and supply the heated air into the reaction vessel. The hot blower 47 may be embodied by a heat exchanger comprising an electric heater such as a nichrome wire. The operation of the hot-air blower 47 prevents the possibility of, when unheated external air is introduced into the chamber in a pressure increase stage after discharging of hydrogen peroxide vapor, the external air condenses the hydrogen peroxide and water vapor. If water vapor is condensed according to the introduction of external air, the condensed moisture blocks the diffusion-limited regions of lumens to prevent the penetration of hydrogen peroxide vapor into the lumens. However, the hot-air blower for supplying hot air does not allow condensation, thereby resulting in improved sterilization efficiency.

Positioned at an external surface of the reaction vessel 10, the heater 18 functions to maintain the atmosphere inside the reaction vessel 10 at a temperature from 30 to 60°C. The heater 18 is adapted to prevent the hydrogen peroxide vapor and water vapor from being condensed within the chamber. However, because the heater 18 is not positioned inside the chamber, it has difficulty in effectively heating the inside of the vacuumed reaction vessel.

In accordance with this embodiment, the radiating heating means 50 is provided for solving this problem. The inner space of the reaction vessel 10 is maintained at low pressures upon vacuumation and sterilization processes. In this state, convection by the heater 18 cannot achieve effective heat transfer and thermal equilibrium in the inner space of the chamber. For example, when the wall of the chamber is heated to about 60°C by the heater 18, the temperature of the atmosphere of the chamber reaches only 20-40°C, with the article maintained at lower temperatures.

The radiating heating means 50 of FIG. 7 can increase the temperature inside the chamber to the same level as that of the inner wall. Thus, the radiating heating means 50 heats hydrogen peroxide vapor, water vapor and articles inside the chamber by radiation, thereby completely removing the possibility of vapor condensation. Particularly, radiated heat enjoys the advantage of uniformly heating all articles irrespective of their number or volume if they are transparent.

Additionally, if the inner wall of the reaction vessel 10 is made from or coated with metal, it reflects heat energy or thermal rays so as to evenly heat the inside of the reaction vessel. An infrared lamp or halogen lamp is used as the radiating heating means 50. Of course, any heater may be employed if it heats in a radiating manner.

In this embodiment, the radiating heating means 50 comprises a lamp 52 and a window 54, such as quartz, for separating the lamp 52 from the inner space of the reaction vessel. In addition, a reflector 56 is preferably provided at the back of the lamp 52 so as to condense the light emitted backwards. In accordance with this embodiment, a proper number of the radiating heating means 50 may be installed on the inner wall of the reaction vessel.

Optionally, the radiating heating means 50 may be operated in an intermittent ON/OFF cycle in order to prevent the articles from being overheated.

Equipped with the heating means described above, the sterilizer of the present invention can prevent the blockage phenomenon attributed to the fact that hydrogen peroxide vapor cannot penetrate into diffusion-limited regions, such as lumens, as water vapor is condensed at inlets of the regions.

### Industrial Applicability

In accordance with the present invention, as described above, the evaporator installed inside the chamber decreases the quantity of an aqueous hydrogen peroxide solution required for sterilization. In addition, the built-in evaporator results in a decrease in the distance between the vapor supply and the articles, bringing about an improvement in sterilization efficiency.

Additionally, the introduction of external air into the chamber during sterilization with hydrogen peroxide vapor facilitates the penetration of the sterilant into diffusion-limited regions, such as lumens, and thus increases the sterilization efficiency.

Further, the decrease in temperature inside the reaction vessel, attributed to the introduction of cool external air, and the resulting condensation of water vapor can be prevented by heating the external air in advance of the introduction using a heater, so that the hydrogen peroxide vapor readily penetrates into diffusion-limited regions.

Moreover, the radiating heating means, installed inside the reaction vessel, directly heats hydrogen peroxide vapor, water vapor, and articles, thereby preventing the condensation of water vapor and improving the sterilizing power of hydrogen peroxide vapor.

## Claims

1. A method for sterilizing an article (11) in a reaction vessel (10) using hydrogen peroxide vapor as a sterilant, comprising:
maintaining an inner space of the reaction vessel (10) at a vacuum pressure lower than an equilibrium vapor pressure of hydrogen peroxide;
heating the inner space of the reaction vessel (10) and an evaporator (40) provided on an internal bottom of the reaction vessel (10) tc a temperature between the range from 30 to 60 °C;
injecting a hydrogen peroxide solution into the inner space of the reaction vessel (10);
thermally vaporizing the hydrogen peroxide solution within the reaction vessel (10) at the same time of the injection step;
sealing the inner space of the reaction vessel (10) and maintaining the article in a hydrogen peroxide vapor atmosphere; and **characterized by**
introducing external gas into the inner space of the reaction vessel (10) to increase and maintain the pressure of the inner space to a predetermined value between the range from 13.33 kPa to 79.99 kPa (100 to 600 Torr).

2. The method according to claim 1, further comprising heating the external gas before the introducing step.

3. The method according to claim 1, wherein the heating step comprises radiating the inner space of the reaction vessel (10) with a radiating heating means (50).

4. The method according to claim 3, wherein the radiating heating means (50) is an infrared lamp or a halogen lamp.

5. The method according to claim 3, wherein the radiating heating means (50) radiates heat energy in an intermittent manner.

6. The method according to claim 1, wherein the introducing step is conducted multiple times to increase the pressure of the inner space in a stepwise manner.

7. The method according to any of claims 1 to 6, further comprising:
decreasing the pressure of the inner space to the predetermined vacuum pressure of the maintaining step or less; and
re-increasing the pressure of the inner space to the atmospheric pressure or less, after the introducing step.

8. The method according to claim 7, further comprising: repeating pressure decrease and pressure re-increase at least once more, after the re-increasing step.

9. The method according to any one of claims 1 to 8, wherein the external gas is air.

## Patentansprüche

1. Verfahren zur Sterilisierung eines Artikels (11) in einem Reaktionsgefäß (10) mittels Wasserstoffperoxiddampf als Sterilisationsmittel:
bei dem ein Innenraum des Reaktionsgefäßes (10) bei einem Vakuum-Druck unterhalb eines Gleichgewichtsdampfdrucks des Wasserstoffperoxids gehalten wird;
bei dem der Innenraum des Reaktionsgefäßes (10) und ein an einem Innenboden des Reaktionsgefäßes (10) angebrachter Verdampfer (40) auf eine Temperatur im Bereich von 30 bis 60 °C erwärmt werden;
bei dem eine Wasserstoffperoxidlösung in den Innenraum des Reaktionsgefäßes (10) injiziert wird;
bei dem die Wasserstoffperoxidlösung während des Injektionsschrittes innerhalb des Reaktionsgefäßes (10) thermisch verdampft wird;
bei dem der Innenraum des Reaktionsgefäßes (10) abdichtet und der Artikel in einer Wasserstoffperoxid-Damptatmosphäre gehalten wird;
**gekennzeichnet durch**
die Einbringung eines externen Gases in den Innenraum des Reaktionsgefäßes (10), um den Druck des Innenraumes auf einen zuvor bestimmten Wert im Bereich von 13,33 kPa bis 79,99 kPa (100 bis 600 Torr) zu erhöhen und dort zu halten.

2. Verfahren gemäß Anspruch 1, bei dem darüber hinaus das externe Gas vor dem Schritt der Einbringung erwärmt wird.

3. Verfahren gemäß Anspruch 1, wobei der Schritt der Erwärmung die Ausstrahlung des Innenraumes des Reaktionsgefäßes (10) mit einem Wärmestrahler (50) umfasst.

4. Verfahren gemäß Anspruch 3, wobei der wärmestrahler (50) eine Infrarot- oder eine Halogenlampe ist.

5. Verfahren gemäß Anspruch 3, wobei der Wärmestrahler (50) die Wärmeenergie intermittierend abgibt.

6. Verfahren gemäß Anspruch 1, wobei der Schritt der Einbringung mehrere Male ausgeführt wird, um den Druck des Innenraumes schrittweise zu erhöhen.

7. Verfahren gemäß einem der Ansprüche 1 bis 6, in welcher darüber hinaus:
der Druck des Innenraums auf den zuvor bestimmten Vakuum-Druck des Schrittes der Aufrechterhaltung oder darunter verringert wird; und
der Druck des Innenraumes nach dem Schritt der Einbringung wiederholt auf atmosphärischen Druck oder darunter erhöht wird.

8. Verfahren gemäß Anspruch 7, bei dem darüber hinaus die Druckverringerung und die erneute Erhöhung des Druckes mindestens ein Mal nach dem Schritt der erneuten Erhöhung wiederholt werden.

9. Verfahren gemäß einem der Ansprüche 1 bis 8, wobei das externe Gas Luft ist.

## Revendications

1. Procédé de stérilisation d'un article (11) dans une cuve de réaction (10) utilisant de la vapeur de peroxyde d'hydrogène comme stérilisant comprenant :
le maintien d'un espace interne de la cuve de réaction (10) à une pression négative inférieure à une pression de vapeur saturante du peroxyde d'hydrogène;
le chauffage de l'espace interne de la cuve de réaction (10) et d'un évaporateur (40) fourni sur un fond interne de la cuve de réaction (10) à une température située entre 30 et 60ºC ;
l'injection d'une solution de peroxyde d'hydrogène dans l'espace interne de la cuve de réaction (10) ;
la vaporisation thermique de la solution de peroxyde d'hydrogène à l'intérieur de la cuve de réaction (10) au moment de l'étape d'injection ;
le scellage de l'espace interne de la cuve de réaction (10) et le maintien de l'article dans une atmosphère de vapeur de peroxyde d'hydrogène ; et caractérisée en
l'introduction d'un gaz externe dans l'espace interne de la cuve de réaction (10) pour augmenter et maintenir la pression de l'espace interne à une valeur prédéterminée située entre 13,33 kPa et 79,99 kPa (100 à 600 Torr).

2. Procédé selon la revendication 1, comprenant en outre le chauffage du gaz externe avant l'étape d'introduction.

3. Procédé selon la revendication 1, selon laquelle l'étape de chauffage comprend l'irradiation de l'espace interne de la cuve de réaction (10) avec des moyens de chauffage rayonnant (50).

4. Procédé selon la revendication 3, selon laquelle les moyens de chauffage rayonnant (50) sont une lampe à infrarouge ou une lampe à halogène.

5. Procédé selon la revendication 3, selon laquelle les moyens de chauffage rayonnant (50) émettent de l'énergie thermique par rayonnement de façon intermittente.

6. Procédé selon la revendication 1, selon laquelle l'étape d'introduction est effectuée plusieurs fois pour augmenter la pression de l'espace interne de façon progressive.

7. Procédé selon l'une quelconque des revendications 1 à 6, comprenant en outre :
la diminution de la pression de l'espace interne à la pression négative prédéterminée de l'étape de maintien ou moins ; et
la ré-augmentation de la pression de l'espace interne à la pression atmosphérique ou moins, après l'étape d'introduction.

8. Procédé selon la revendication 7, comprenant en outre : la répétition de la diminution de pression et de la ré-augmentation de pression au moins une fois de plus, après l'étape de ré-augmentation.

9. Procédé selon l'une quelconque des revendications 1 à 8, selon laquelle le gaz externe est de l'air.
